**Europäisches Patentamt**

**European Patent Office**

**Office européen des brevets**

(19)

(11) Veröffentlichungsnummer: **0 166 685**
**B1**

(12)

# EUROPÄISCHE PATENTSCHRIFT

(45) Veröffentlichungstag der Patentschrift:
**28.12.88**

(51) Int. Cl.⁴: **C 07 C 102/00, C 07 C 103/34**

(21) Anmeldenummer: **85810243.7**

(22) Anmeldetag: **23.05.85**

(54) Verfahren zur Herstellung von Alpha-Chloracetessigsäuremonomethylamid.

(30) Priorität: **29.05.84 CH 2636/84**

(43) Veröffentlichungstag der Anmeldung:
**02.01.86 Patentblatt 86/1**

(45) Bekanntmachung des Hinweises auf die Patenterteilung:
**28.12.88 Patentblatt 88/52**

(84) Benannte Vertragsstaaten:
**AT BE CH DE FR GB IT LI LU NL SE**

(56) Entgegenhaltungen:
**EP-A- 0 005 849**
**GB-A- 2 000 499**

(73) Patentinhaber: **CIBA-GEIGY AG, Klybeckstrasse 141, CH-4002 Basel (CH)**

(72) Erfinder: **Rigamonti, Flaviano, Dr., Chemin de Champerfou 75A, CH-1870 Monthey (CH)**
Erfinder: **Raghunandan, Pullissery, Fiat No. S-4 Madhuban-2 Sta. Inez, Panaji Goa 403001 (IN)**

ACTORUM AG

## Beschreibung

Die vorliegende Erfindung betrifft ein Verfahren zur Herstellung von α-Chloracetessigsäuremonomethylamid durch Chlorierung von Acetessigsäuremonomethylamid in wässrigem Medium.

α-Chloracetessigsäuremonomethylamid ist ein wertvolles Zwischenprodukt zur Herstellung von insektiziden Wirkstoffen. Beispielsweise liefert es bei der Umsetzung mit Trimethylphosphit 0,0-Dimethyl-0-(1-methyl-2-methylcarbamoylvinyl)-phosphat, das unter der Bezeichnung Monocrotophos als Wirkstoff in insektiziden Formulierungen bekannt geworden ist.

Bei der Herstellung von α-Chloracetessigsäuremonomethylamid durch Chlorierung von Acetessigsäuremonomethylamid in wässrigem Medium und der Abtrennung des Produkts in fester Form treten im wesentlichen zwei Probleme auf. Das eine besteht darin, dass infolge der hohen Reaktivität der α-Methylengruppe des Acetessigsäuremonomethylamids leicht beide α-ständigen Wasserstoffe durch Chlor ersetzt werden. Das andere wird durch die Löslichkeit von α-Chloracetessigsäuremonomethylamid in Wasser verursacht, welche die vollständige Abtrennung des Produkts aus dem wässrigen Reaktionsmedium erschwert.

Es wurde bereits vorgeschlagen, die α, α-Dichlorierung dadurch zu vermeiden, dass man die Chlorierung bei tiefen Temperaturen in Gegenwart von Harnstoff und unter Verwendung von weniger als der stöchiometrischen Menge Chlor durchführte (vgl. US-P 3 483 252 und 4 235 818). Die durch die hohe Wasserlöslichkeit des α-Chloracetessigsäuremonomethylamids verursachten Ausbeuteverluste versuchte man dadurch gering zu halten, dass man jeweils 50-85% der nach der Abtrennung von festem α-Chloracetessigsäuremonomethylamid erhaltenen Mutterlauge in den nächsten Ansatz zurückführte (vgl. US-P 4 235 818).

Die mit der partiellen Rückführung der Mutterlauge aus dem vorangehenden Ansatz erreichbaren Ergebnisse sind jedoch weder in technischer noch in ökonomischer und ökologischer Hinsicht voll befriedigend, da einerseits die insbesondere bei mehrmaliger Rückführung von Mutterlauge erfolgende Anreicherung von Nebenprodukten eine Verschlechterung der Produktqualität zur Folge hat und andererseits jeweils 15-50% der Mutterlauge mit dem darin noch enthaltenen Acetessigsäuremonomethylamid (etwa 3 Gew.-%) und α-Chloracetessigsäuremonomethylamid (etwa 4 Gew.-%) verloren gehen und die Umwelt belasten.

Es war daher die Aufgabe der vorliegenden Erfindung, diese Mängel zu beseitigen, und ein Verfahren bereitzustellen, dass eine möglichst verlustfreie und umweltfreundliche Herstellung von α-Chloracetessigsäuremonomethylamid von guter Qualität ermöglicht.

Zur Lösung dieser Aufgabe wird erfindungsgemäss vorgeschlagen, die Herstellung von α-Chloracetessigsäuremonomethylamid durch Einwirkung von Chlor auf Acetessigsäuremonomethylamid in wässrigem Medium bei Temperaturen von −20 bis +10°C in Gegenwart von Harnstoff und Alkalisalzen, Neutralisierung des Reaktionsgemisches, Abtrennung von festem α-Chloracetessigsäuremonomethylamid und Rückführung der Mutterlauge in die Chlorierungsreaktion in der Weise durchzuführen, dass man die jeweils nach der Abtrennung von α-Chloracetessigsäuremonomethylamid erhaltene Mutterlauge vor ihrer Rückführung mit 2-10 Gew.-% bezogen auf die Gesamtmenge der Mutterlauge eines inerten, mit Wasser nicht mischbaren Lösungsmittels extrahiert.

Geeignete Lösungsmittel, die für die Extraktion der Mutterlauge in Betracht kommen, sind aliphatische und aromatische Kohlenwasserstoffe, wie Hexan, Heptan, Cyclohexan, Benzol, Toluol und Xylol, ferner halognierte aliphatische und aromatische Kohlenwasserstoffe, wie Methylenchlorid, Chloroform, Kohlenstofftetrachlorid, 1,2-Dichloräthan, Chlorbenzol und o-Dichlorbenzol. Besonders geeignete Lösungsmittel sind 1,2-Dichloräthan und Toluol.

Die Extraktion kann sowohl batchweise ein- oder mehrstufig in Rührkesseln oder aber auch kontinuierlich in Extraktionskolonnen mit 1-10, vorzugsweise 3-5 theoretischen Trennstufen durchgeführt werden. Bei der kontinuierlichen Durchführung kann sowohl nach dem Gleich- als auch nach dem Gegenstromprinzip gearbeitet werden. Wegen der korrosiven Eigenschaften der Mutterlauge verwendet man vorteilhaft Apparaturen aus Glas, Email oder hochlegierten Stählen wie z. B. Hastelloy C.

Die Menge an Extraktionsmittel liegt in der Regel bei 2-10 Gew.-% bezogen auf die Gesamtmenge an zu extrahierender Mutterlauge. Für die einstufige Extraktion in Rührkesseln verwendet man vorzugsweise 4-6 Gew.-% Lösungsmittel und für die mehrstufige Extraktion vorzugsweise 3-5 Gew.-% Lösungsmittel bezogen auf die Gesamtmenge der zu extrahierenden Mutterlauge. Die Extraktion kann im Temperaturbereich von −20-50°C durchgeführt werden und nimmt 15 Minuten bis 2 Stunden in Anspruch. Vorzugsweise wird die Extraktion bei 0-20°C durchgeführt.

Nach der Extraktion kann die Mutterlauge ohne weitere Reinigung direkt in den Chlorierungsprozess zurückgeführt werden. Das zur Extraktion verwendete Lösungsmittel kann aus dem Extrakt durch Destillation zurückgewonnen und wieder verwendet werden. Der Destillationsrückstand enthält neben α-Dichloracetessigsäuremonomethylamid und Acetessigsäuremonomethylamid auch α-Chloracetessigsäuremonomethylamid. Diese Substanzen können aus dem Destillationsrückstand durch fraktionierte Kristallisation aus Toluol oder 1,2-Dichloräthan gewonnen werden. Das im Destillationsrückstand enthaltene α-Chloracetessigsäuremonomethylamid wird auf diese Weise zu 50-70% erhalten.

Die erfindungsgemässe Extraktion der Mutterlauge ermöglicht im Gegensatz zum bekannten Verfahren die Rückführung der gesamten Mutterlauge. Damit können die bei der Herstellung von α-Chloracetessigsäuremonomethylamid auftretenden Verluste auf ein Minimum reduziert werden. Gleichzeitig wird das α-Chloracetessigsäuremonomethylamid in höherer Reinheit und in besserer Ausbeute erhalten als mit bekannten Verfahren. Wegen der Rückführbarkeit der gesamten Mutterlauge kann zur Vermeidung

der Bildung von α, α-Chloracetessigsäuremonomethylamid ohne weiteres weniger als die äquivalente Menge Chlor, beispielsweise 0,75-0,90 Mol Chlor pro Mol Acetessigsäuremonomethylamid verwendet werden, ohne dass nicht umgesetztes Acetessigsäuremonomethylamid, wie beim bekannten Verfahren mit der ausgekreisten Mutterlauge verloren geht. Dieses ingesamt vorteilhafte Ergebnis des erfindungsgemässen Verfahrens muss als überraschend angesehen werden, denn es war nicht vorauszusehen, dass bei der Extraktion der Mutterlauge mit den obengenannten Lösungsmitteln die darin enthaltenen Nebenprodukte, die Ausbeute und Produktqualität nachteilig beeinflussen, selektiv entfernt werden können.

Das erfindungsgemässe Verfahren wird durch die nachfolgenden Beispiele näher erläutert.

*Beispiel 1*
Herstellung von α-Chloracetessigsäuremonomethylamid mit Mutterlauge-Rückführung ohne Extraktion (Stand der Technik)

In einem Rührkessel werden 1560 kg einer 50%-igen wässrigen Lösung von Acetessigsäuremonomethylamid (780 kg Acetessigsäuremonomethylamid; 6,76 kMol), 78 kg 1,2-Dichloräthan, 181 kg Wasser, 206 kg Natriumchlorid, 131 kg Harnstoff und 6045 kg Mutterlauge aus dem vorhergehenden Ansatz, enthaltend

15   Gew.-% Natriumchlorid
8    Gew.-% Harnstoff
3    Gew.-% Acetessigsäuremonomethylamid
4    Gew.-% α-Chloracetessigsäuremonomethylamid
3    Gew.-% Nebenprodukte, insbesondere α, α-Dichloracetessigsäuremonomethylamid
3,5  Gew.-% Ammoniumchlorid
2    Gew.-% 1,2-Dichloräthan
61,5 Gew.-% Wasser

vorgelegt. In diese Mischung werden unter Niveau bei −16 bis −17°C 475 kg (6,7 kMol) Chlor eingeleitet. Anschliessend wird das Reaktionsgemisch bei −6°C durch Zugabe von 114 kg Ammoniak auf pH 6,5-6,8 gestellt und das abgeschiedene α-Chloracetessigsäuremonomethylamid durch Zentrifugieren abgetrennt. Es werden so 814 kg α-Chloracetessigsäuremonomethylamid (83%-ig; 67% der Theorie) und 7502 kg Mutterlauge erhalten, von der 80% (6045 kg) in die nächste Umsetzung zurück geführt werden. Der Rest der Mutterlauge wird verworfen.

*Beispiel 2*
Herstellung von α-Chloracetessigsäuremonomethylamid mit Rückführung von extrahierter Mutterlauge (erfindungsgemässes Verfahren)

a) *Extraktion der Mutterlauge*

8405 kg Mutterlauge der Zusammensetzung
16   Gew.-% Natriumchlorid
8    Gew.-% Harnstoff
3    Gew.-% Acetessigsäuremonomethylamid
4    Gew.-% α-Chloracetessigsäuremonomethylamid
3    Gew.-% Nebenprodukte, insbesondere α, α-Dichloracetessigsäuremonomethylamid
3,5  Gew.-% Ammoniumchlorid
2,0  Gew.-% 1,2-Dichloräthan
60,5 Gew.-% Wasser

werden in einem Rührkessel bei Raumtemperatur durch intensives Rühren eine Stunde mit 450 kg 1,2-Dichloräthan vermischt. Dann wird die Rührung unterbrochen und die Schichten werden getrennt.

b) *Rückführung der extrahierten Mutterlauge*

In einem Rührkessel werden 960 kg einer 85%igen wässrigen Lösung von Acetessigsäuremonomethylamid (780 kg Acetessigsäuremonomethylamid; 6,76 kMol), 25 kg Harnstoff, 104 kg 1,2-Dichloräthan und 8320 kg der nach a) erhaltenen Mutterlauge vorgelegt. In diese Mischung werden bei −16 bis −17°C 520 kg (7,33 kMol) Chlor unter Niveau eingeleitet. Anschliessend wird der pH-Wert des Reaktionsgemisches bei −6°C durch Zugabe von 125 kg Ammoniak auf 6,5-6,8 gestellt und das abgeschiedene α-Chloracetessigsäuremonomethylamid durch Zentrifugieren abgetrennt. Es werden so 839 kg α-Chloracetessigsäuremonomethylamid (91%ig; 75% der Theorie bezogen auf frisch eingesetztes Acetessigsäuremonomethylamid) und 8577 kg Mutterlauge erhalten, die nach Extraktion mit 1,2-Dichloräthan in den nächsten Ansatz zurückgeführt wird.

**Patentansprüche**

1. Verfahren zur Herstellung von α-Chloracetessigsäuremonomethylamid durch Einwirkung von Chlor auf Acetessigsäuremonomethylamid in wässrigem Medium bei Temperaturen von −20 bis +10°C in Gegenwart von Harnstoff und Alkalisalzen, Neutralisierung des Reaktionsgemisches, Abtrennung von festem α-Chloracetessigsäuremonomethylamid und Rückführung der Mutterlauge in die Chlorierungsreaktion, dadurch gekennzeichnet, dass man die jeweils nach der Abtrennung von α-Chloracetessigsäuremonomethylamid erhaltene Mutterlauge vor ihrer Rückführung mit 2-10 Gew.-% bezogen auf die Gesamtmenge der Mutterlauge eines inerten, mit Wasser nicht mischbaren Lösungsmittels extrahiert.

2. Verfahren nach Anspruch 1, dadurch gekennzeichnet, dass man als inertes, mit Wasser nicht mischbares Lösungsmittel einen aliphatischen oder aromatischen Kohlenwasserstoff oder einen halogenierten aliphatischen oder aromatischen Kohlenwasserstoff verwendet.

3. Verfahren nach Anspruch 2, dadurch gekennzeichnet, dass man als inertes, mit Wasser nicht mischbares Lösungsmittel Hexan, Heptan, Cyclohexan, Benzol, Toluol, Xylol, Methylenchlorid, Chloroform, Kohlenstofftetrachlorid, 1,2-Dichloräthan, Chlorbenzol oder ein o-Dichlorbenzol verwendet.

4. Verfahren nach Anspruch 3, dadurch gekennzeichnet, dass man als inertes, mit Wasser nicht mischbares Lösungsmittel Toluol oder 1,2-Dichloräthan verwendet.

5. Verfahren nach Anspruch 1, dadurch gekennzeichnet, dass man in einer einstufigen Extraktion 4-6 Gew.-% Lösungsmittel bezogen auf die Gesamtmenge an zu extrahierender Mutterlauge verwendet.

6. Verfahren nach Anspruch 1, dadurch gekennzeichnet, dass man bei der mehrstufigen Extraktion 4-5 Gew.-% Lösungsmittel bezogen auf die Gesamtmenge an zu extrahierender Mutterlauge verwendet.

7. Verfahren nach Anspruch 1, dadurch gekennzeichnet, dass man die Extraktion im Temperaturbereich von −20 bis 50°C durchführt.

8. Verfahren nach Anspruch 7, dadurch gekennzeichnet, dass man die Extraktion bei 0-20°C durchführt.

## Claims

1. A process for producing α-chloroacetoacetic acid monomethylamide by reaction of chlorine with acetoacetic acid monomethylamide in an aqueous medium, at temperatures of −20 to +10°C, in the presence of urea and alkali metal salts, neutralisation of the reaction mixture, separation of solid α-chloroacetoacetic acid monomethylamide, and feeding back of the mother liquor into the chlorination reaction, wherein the mother liquor obtained each time after the separation of α-chloroacetoacetic acid monomethylamide is extracted, before being recycled, with 2-10% by weight, relative to the total amount of the mother liquor, of an inert solvent immiscible with water.

2. A process according to claim 1, wherein the inert water-immiscible solvent used is an aliphatic or aromatic hydrocarbon or a halogenated aliphatic or aromatic hydrocarbon.

3. A process according to claim 2, wherein the inert water-immiscible solvent used is hexane, heptane, cyclohexane, benzene, toluene, xylene, methylene chloride, chloroform, carbon tetrachloride, 1,2-dichloroethane, chlorobenzene or o-dichlorobenzene.

4. A process according to claim 3, wherein the inert water-immiscible solvent used is toluene or 1,2-dichloroethane.

5. A process according to claim 1, wherein there is used, for a single-stage extraction, 4-6% by weight of solvent, relative to the total amount of mother liquor to be extracted.

6. A process according to claim 1, wherein there is used, for a multistage extraction, 4-5% by weight of solvent, relative to the total amount of mother liquor to be extracted.

7. A process according to claim 1, wherein the extraction is performed in the temperature range of −20 to 50°C.

8. A process according to claim 7, wherein the extraction is performed at 0-20°C.

## Revendications

1. Procédé de préparation du monométhylamide de l'acide α-chloracétylacétique par action du chlore sur le monométhylamide de l'acide acétylacétique en milieu aqueux à des températures de −20 à +10°C en présence d'urée et de sels alcalins, neutralisation du mélange de récaction, séparation du monométhylamide solide de l'acide α-chloracétylacétique et recyclage des liqueurs-mères à la réaction de chloration, caractérisé en ce que les liqueurs-mères obtenues après séparation du monométhylamide de l'acide α-chloracétylacétique sont extraites à chaque fois, avant d'être recyclées, par un solvant inerte, non miscible à l'eau, en quantité de 2 à 10% en poids, par rapport à la quantité totale des liqueurs-mères.

2. Procédé selon la revendication 1, caractérisé en ce que l'on utilise en tant que solvant inerte, non miscible à l'eau, un hydrocarbure aliphatique ou aromatique ou un hydrocarbure aliphatique ou aromatique halogéné.

3. Procédé selon la revendication 2, caractérisé en ce que l'on utilise en tant que solvant inerte non miscible à l'eau l'hexane, l'heptane, le cyclohexane, le benzène, le toluène, le xylène, le chlorure de méthylène, le chloroforme, le tétrachlorure de carbone, le 1,2-dichloréthane, le chlorobenzène ou un o-dichlorobenzène.

4. Procédé selon la revendication 3, caractérisé en ce que l'on utilise en tant que solvant inerte non miscible à l'eau le toluène ou le 1,2-dichloréthane.

5. Procédé selon la revendication 1, caractérisé en ce que, dans une extraction en un seul stade opératoire, on utilise le solvant en quantité de 4 à 6% en poids par rapport à la quantité totale des liqueurs-mères à extraire.

6. Procédé selon la revendication 1, caractérisé en ce que, dans une extraction en plusieurs stades opératoires, on utilise le solvant en quantité de 4 à 5% en poids par rapport à la quantité total des liqueurs-mères à extraire.

7. Procédé selon la revendication 1, caractérisé en ce que l'on effectue l'extraction dans un intervalle de température de −20 à +50°C.

8. Procédé selon la revendication 7, caractérisé en ce que l'on effectue l'extraction entre 0 et 20°C.